(19) Europäisches Patentamt
European Patent Office
Office européen des brevets

(11) EP 4 209 480 A1

(12) **EUROPEAN PATENT APPLICATION**
published in accordance with Art. 153(4) EPC

(43) Date of publication:
**12.07.2023 Bulletin 2023/28**

(21) Application number: **21864294.0**

(22) Date of filing: **30.08.2021**

(51) International Patent Classification (IPC):
***C07C 51/00*** *(2006.01)* ***C07C 51/02*** *(2006.01)*
***C07C 53/02*** *(2006.01)* ***C07C 53/06*** *(2006.01)*
***C07F 15/00*** *(2006.01)* ***C07F 19/00*** *(2006.01)*
***C09K 3/00*** *(2006.01)* ***C07B 61/00*** *(2006.01)*
***C07F 9/50*** *(2006.01)* ***B01J 31/02*** *(2006.01)*
***B01J 31/22*** *(2006.01)* ***B01J 31/24*** *(2006.01)*

(52) Cooperative Patent Classification (CPC):
**B01J 31/02; B01J 31/22; B01J 31/24; C07B 61/00; C07C 51/00; C07C 51/02; C07C 53/02; C07C 53/06; C07F 9/50; C07F 15/00; C07F 19/00; C09K 3/00**

(86) International application number:
**PCT/JP2021/031793**

(87) International publication number:
**WO 2022/050236 (10.03.2022 Gazette 2022/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.09.2020 JP 2020148562**
**12.02.2021 JP 2021021223**
**12.02.2021 JP 2021021224**
**12.02.2021 JP 2021021225**
**10.05.2021 JP 2021079887**
**17.05.2021 JP 2021083416**

(71) Applicant: **NITTO DENKO CORPORATION**
**Ibaraki-shi**
**Osaka 567-8680 (JP)**

(72) Inventors:
- **HIRANO Makoto**
  **Ibaraki-shi, Osaka 567-8680 (JP)**
- **MATSUDA Hirokazu**
  **Ibaraki-shi, Osaka 567-8680 (JP)**
- **PIDKO, Evgeny Alexandrovich**
  **3584 HT Utrecht (NL)**
- **FILONENKO, Georgy Alexandrovich**
  **2613 VG Delft (NL)**
- **REBREYEND, Christophe**
  **1062 KS Amsterdam (NL)**

(74) Representative: **Hoffmann Eitle**
**Patent- und Rechtsanwälte PartmbB**
**Arabellastraße 30**
**81925 München (DE)**

# (54) PRODUCTION METHOD FOR ALKALINE EARTH METAL FORMATE

(57) The present invention relates to a production method for an alkaline earth metal formate, said method including a first step for producing a formate of an alkaline earth metal by reacting hydrogen, carbon dioxide, and a carbonate or bicarbonate of an alkaline earth metal using a homogeneous catalyst in the presence of a solvent in a two-phase system in which the solvent is present in a state in which the solvent has been separated into an organic phase and a water phase.



Processed by Luminess, 75001 PARIS (FR)

## Description

TECHNICAL FIELD

The present invention relates to a method for producing an alkaline earth metal formate.

BACKGROUND ART

Formates of alkaline earth metals have been traditionally used in various applications.
For example, calcium formate is used for neutralizing agents in leather production, curing accelerators for cement, livestock feed additives, and the like. As a method for producing calcium formate, various methods such as a method of reacting formic acid with calcium hydroxide and a method of causing carbon monoxide to act on calcium hydroxide are known.
Patent Literature 1 describes a method for producing fragment-shaped calcium formate in which particles have a particle size in the range of 0.2 to 5 mm, from microparticulate or powdered calcium formate having an average particle size of 0.2 mm or less.
On the other hand, from the standpoint of the problems of global warming and the like, technologies for converting carbon dioxide into useful compounds have been examined.
For example, in Non-Patent Literatures 1 and 2, methods for producing a formate from carbon dioxide ($CO_2$) and hydrogen ($H_2$) in the presence of a catalyst are examined.

CITATION LIST

PATENT LITERATURE

Patent Literature 1: JPH11-286466A

NON-PATENT LITERATURES

Non-Patent Literature 1: Hydrogenation of aqueous mixtures of calcium carbonate and carbon dioxide using a water-soluble rhodium (I)-tertiary phosphine complex catalyst (https://www.sciencedirect.com/science/article/abs/pii/S1381116904006338)
Non-Patent Literature 2: Eco-friendly Upconversion of Limestone into Value-added Calcium Formate (https://pubs.rsc.org/en/content/articlelanding/2020/gc/d0gc01089h#!divAbstract)

SUMMARY OF INVENTION

TECHNICAL PROBLEM

The technology described in Non-Patent Literature 1 is such that a formate is formed from hydrogen and carbon dioxide using a metal complex catalyst of a uniform system. However, since the reaction is conducted in a one-phase system, there is a risk of deterioration of the catalyst activity and decomposition of the formate when the formate is separated from the catalyst and the solvent or the solvent is distilled off. Furthermore, the technology described in Non-Patent Literature 2 is such that a formate is formed from hydrogen and carbon dioxide using a heterogeneous metal complex catalyst, and a method in which reactivity is insufficient and a formate can be formed in higher yield is required.
Thus, the invention provides a method for producing an alkaline earth metal formate, which can produce an alkaline earth metal formate in high yield and with excellent productivity.

SOLUTION TO PROBLEM

As a result of intensive investigations, the present inventors have found a method for producing an alkaline earth metal formate, in which an alkaline earth metal formate can be produced in high yield and with excellent productivity by conducting a reaction in a two-phase system using a homogeneous catalyst, and have completed the invention.
Means for solving the above problems are as follows.

[1] A method for producing an alkaline earth metal formate, the method comprising:

a first step of reacting hydrogen and carbon dioxide with a carbonate or hydrogen carbonate of an alkaline earth metal
using a homogeneous catalyst
in the presence of a solvent
in a two-phase system in which an organic phase and an aqueous phase are present in a separated state in the solvent
to produce a formate of an alkaline earth metal.

[2] The method for producing an alkaline earth metal formate described in [1], wherein the method further includes a second step of separating a solution including the homogeneous catalyst from the reaction solution obtained by the first step, by partitioning.
[3] The method for producing an alkaline earth metal formate described in [1] or [2], wherein the alkaline earth metal is calcium.
[4] The method for producing an alkaline earth metal formate described in any one of [1] to [3], wherein the homogeneous catalyst contains at least one metal selected from ruthenium, iridium, iron, nickel, and cobalt.
[5] The method for producing an alkaline earth metal formate described in any one of [1] to [4], wherein the organic phase contains at least one selected from toluene, dioxane, tetrahydrofuran, ethyl acetate, methylcyclohexane, and cyclopentyl methyl ether.
[6] The method for producing an alkaline earth metal formate described in any one of [1] to [5], wherein the homogeneous catalyst is a metal complex catalyst, and a ligand of the metal complex catalyst is further added.
[7] The method for producing an alkaline earth metal formate described in any one of [1] to [5], wherein the homogeneous catalyst is at least one selected from a ruthenium complex represented by the following formula (1), a tautomer or stereoisomer thereof, and a salt compound of the ruthenium complex, tautomer or stereoisomer:

[Chem. 1]

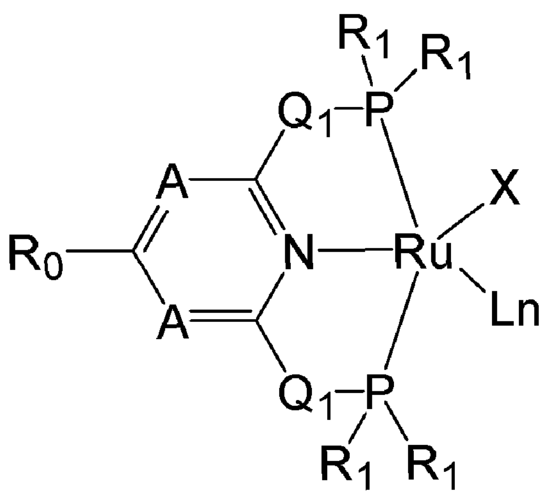

(1)

(in the formula (1), $R_0$ represents a hydrogen atom or an alkyl group,
$Q_1$ each independently represents $CH_2$, NH or O,
$R_1$ each independently represents an alkyl group or an aryl group (provided that when $Q_1$ represents NH or O, at least one of $R_1$ represents an aryl group),
A each independently represents CH, $CR_5$ or N, $R_5$ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.)

[8] The method for producing an alkaline earth metal formate described in [7], wherein a ligand represented by the following formula (4) is further added:

[Chem. 2]

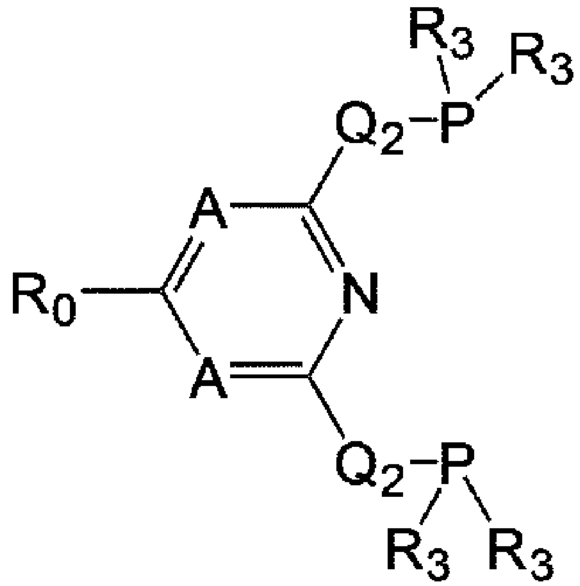

(4)

(In the formula (4), $R_0$ represents a hydrogen atom or an alkyl group,
$Q_2$ each independently represents NH or O,
$R_3$ each independently represents an aryl group,
A each independently represents CH, $CR_5$ or N, and $R_5$ represents an alkyl group, an aryl group, an aralkyl group, an amino group, a hydroxy group or an alkoxy group.)

[9] The method for producing an alkaline earth metal formate described in any one of [1] to [8], wherein a quaternary ammonium salt is further used as a phase transfer catalyst in the first step.

ADVANTAGEOUS EFFECTS OF INVENTION

According to the invention, a method for producing an alkaline earth metal formate, in which an alkaline earth metal formate can be produced in high yield and with excellent productivity, can be provided.

DESCRIPTION OF EMBODIMENTS

Embodiments of the invention are described in detail below.

The method for producing an alkaline earth metal formate according to an embodiment of the invention is a method for producing a formate of an alkaline earth metal, which includes a first step of reacting hydrogen and carbon dioxide with a carbonate or hydrogen carbonate of an alkaline earth metal using a homogeneous catalyst in the presence of a solvent in a two-phase system in which an organic phase and an aqueous phase are present in a separated state in the solvent.

In the method for producing an alkaline earth metal formate (hereinafter, may be simply referred to as a formate) according to an embodiment of the invention, the reaction of hydrogen and carbon dioxide with a carbonate or hydrogen carbonate of an alkaline earth metal is preferably conducted in a catalyst solution (organic phase) in which a homogeneous catalyst (hereinafter, may be simply referred to as catalyst) is dissolved in an organic solvent.

Since the formate formed by the reaction dissolves in an aqueous solvent, the formate is eluted into the aqueous phase. Therefore, the reaction that forms the alkaline earth metal formate is prevented from stopping due to equilibrium, and the alkaline earth metal formate can be formed in high yield. Furthermore, since an aqueous solution of the alkaline earth metal formate is used and can be separated from the catalyst solution by a convenient method, the catalyst activity is not easily deactivated, highly expensive catalyst can be reused, and high productivity can be realized.

According to the method for producing an alkaline earth metal formate related to an embodiment of the invention, hydrogen and carbon dioxide can be stored as an alkaline earth metal formate. An alkaline earth metal formate has advantages that an alkaline earth metal formate has a high hydrogen storage density, is safe, can be conveniently handled since it is stable as a chemical substance, and can store hydrogen and carbon dioxide for a long period of time.

An alkaline earth metal formate has high solubility in an aqueous solvent and can be fractionated as a high-concentration aqueous solution of the alkaline earth metal formate so that the alkaline earth metal formate can be recovered as a solid by simple operation such as distillation as necessary.

The reaction in the method for producing an alkaline earth metal formate according to an embodiment of the invention can be conducted, for example, as follows.

A reaction vessel equipped with a stirring device is provided. As necessary, a phase transfer catalyst may be further added. A carbonate or hydrogen carbonate of an alkaline earth metal dissolved in a solvent and a homogeneous catalyst solution dissolved in a solvent are added to the reaction vessel. Hydrogen and carbon dioxide are introduced into the reaction vessel, and a reaction is conducted.

The homogeneous catalyst, the solvent, hydrogen, carbon dioxide, the carbonate or hydrogen carbonate of an alkaline earth metal salt, and the like, which are used for the reaction, will be described below.

(Solvent)

The solvent according to an embodiment of the invention is not particularly limited so long as it can be obtained as a two-phase system in which an organic phase and an aqueous phase are present in a separated state in the reaction solution, and preferably contains a solvent that dissolves the catalyst to form a uniform solution.

The organic phase is a phase that contains an organic solvent as the solvent, and the aqueous phase is a phase that contains an aqueous solvent as the solvent.

The aqueous solvent includes, for example, water, methanol, ethanol, ethylene glycol, glycerin and mixed solvents thereof. Water is preferred from the standpoint of low environmental load.

The organic solvent includes, for example, toluene, benzene, xylene, propylene carbonate, dioxane, dimethyl sulfoxide, tetrahydrofuran, ethyl acetate, methylcyclohexane, cyclopentyl methyl ether and mixed solvents thereof, and at least one selected from toluene, dioxane, tetrahydrofuran, ethyl acetate, methylcyclohexane and cyclopentyl methyl ether is preferred. From the standpoint of separability from the aqueous solvent, the organic solvent is preferably toluene or dioxane. That is, the organic solvent preferably contains at least one selected from toluene, dioxane, tetrahydrofuran, ethyl acetate, methylcyclohexane and cyclopentyl methyl ether, and the organic phase more preferably contains toluene or dioxane.

[Homogeneous catalyst]

The catalyst used in an embodiment of the invention needs to be a homogeneous catalyst and is preferably a catalyst that dissolves in an organic solvent, and more preferably a compound containing a metal element (metal element compound).

The metal element compound includes salts of metal elements with inorganic acids, such as a hydride salt, an oxide salt, a halide salt (chloride salt or the like), a hydroxide salt, a carbonic acid salt, a hydrogen carbonic acid salt a sulfuric acid salt, a nitric acid salt, a phosphoric acid salt, a boric acid salt, a halogen acid salt, a perhalogen acid salt, a halous acid salt, a hypohalous acid salt, and a thiocyanic acid salt; salts of metal elements with organic acids, such as an alkoxide salt, a carboxylic acid salt (an acetic acid salt, a (meth)acrylic acid salt, or the like), and a sulfonic acid salt (a trifluoromethanesulfonic acid salt or the like); salts of metal elements with organic bases, such as an amide salt, a sulfonamide salt, and a sulfonimide salt (a bis(trifluoromethanesulfonyl)imide salt or the like); complex salts such as an acetylacetone salt, a hexafluoroacetylacetone salt, a porphyrin salt, a phthalocyanine salt, and a cyclopentadiene salt; and complexes or salts containing one or more of nitrogen compounds including a chain-like amine, a cyclic amine, an aromatic amine or the like, phosphorous compounds, compounds containing phosphorus and nitrogen, sulfur compounds, carbon monoxide, carbon dioxide, and water. These compounds may be either hydrates or anhydrides and are not particularly limited. Among these, a halide salt, a complex containing a phosphorus compound, a complex containing a nitrogen compound, and a complex or salt containing a compound containing phosphorus and nitrogen are preferred from the standpoint of further increasing the efficiency of forming the alkaline earth metal formate.

These may be used singly, or two or more kinds may be used in combination.

A commercially available product can be used as the metal element compound, or a compound produced by a known method or the like can also be used. As the known method, for example, a method described in JP5896539B and methods described in Chem. Rev. 2017, 117, 9804-9838 and Chem. Rev. 2018, 118, 372-433 can be used.

The catalyst according to an embodiment of the invention is not particularly limited. However, a catalyst having a catalyst turnover number: TON of 10,000 or more as determined by the following calculation method.

The TON is preferably 10,000 or more, more preferably 50,000 or more, and still more preferably 100,000 or more, from the standpoint of suppressing the production cost for the alkaline earth metal formate. Furthermore, since a higher TON is more preferred, the upper limit is not particularly limited and can be set to, for example, 10,000,000 or less.

TON calculation method:

$$TON = X/Y \quad \text{formula 1}$$

(In the formula 1, X represents a molar amount X (mol) of potassium formate formed by the following TON calculation reaction, a value of X is calculated by the following formula 2, and Y represents the molar amount (mol) of the catalyst used in the following reaction.)

$$X = (W/M) \times (Ia \times Ib/R) \times (A/B) \quad \text{formula 2}$$

(In the formula 2, W represents the amount (g) of dimethyl sulfoxide used for the quantification of potassium formate,
M represents the molecular weight of dimethyl sulfoxide,
R represents the ratio of the number of protons of dimethyl sulfoxide to the number of protons of potassium formate,
Ia represents the proton NMR integration value of potassium formate,
Ib represents the proton NMR integration value of dimethyl sulfoxide,
A represents the mass (g) of the aqueous solution in the lower layer obtained by the following reaction, and
B represents the mass (g) of the aqueous solution used for the quantification of potassium formate.)

TON calculation reaction:

(Production of formate)

10 mmol of $KHCO_3$ was added to 1 mL of water in a glass vial equipped with a stirring rod in a glovebox under an inert gas, and then 0.12 μmol of a catalyst and 54 μmol of methyltrioctylammonium chloride were added to 1 mL of a solvent in which the catalyst obtains the highest TON and which is selected from toluene, dioxane, tetrahydrofuran, ethyl acetate, methylcyclohexane, and cyclopentyl methyl ether. Thereafter, the vial was placed in an autoclave, the autoclave was sealed and taken out of the glovebox.
The autoclave was heated to 90°C while being stirred. When the temperature reached the target temperature, the autoclave was pressurized to 4.5 MPa with $H_2$. The reaction mixture was stirred for 18 hours, thereafter the reaction mixture was cooled with an ice bath, and pressure was carefully released.
An upper layer of the solution after the reaction was removed, and A g of an aqueous solution of a lower layer containing potassium formate and unreacted $KHCO_3$ was obtained.

(Quantification of potassium formate)

B g of the aqueous solution of the lower layer was collected and dissolved in 500 μL of deuterated water, W g of dimethyl sulfoxide was added as an internal standard, thereafter $^1$H NMR analysis was performed, and the NMR integration value of potassium formate was designated as Ia, while the NMR integration value of dimethyl sulfoxide was designated as Ib.
For example, TON determined by the above-TON calculation method for the Ru catalyst 1 and Ru catalyst 7 used for the Examples of the invention are 66,000 for the Ru catalyst 1 and 56,000 for the Ru catalyst 7.
The homogeneous catalyst used in the method for producing an alkaline earth metal formate according to an embodiment of the invention preferably contains at least one metal selected from ruthenium, iridium, iron, nickel, and cobalt, and preferably contains ruthenium. Among them, the homogeneous catalyst is preferably at least one selected from a ruthenium complex represented by formula (1), a tautomer or stereoisomer thereof, and a salt compound of the complex, tautomer or stereoisomer.
The ruthenium complex represented by the formula (1) is dissolved in an organic solvent and is insoluble in water. A formate formed in the reaction is easily dissolved in water. Therefore, the separation of the catalyst and the formate is easily achieved by a two-phase system reaction, the catalyst and the formate are respectively easily separated and recovered from the reaction system, and this enabled production of a formate in high yield.
According to the method of the present embodiment, a formate formed in the reaction can be separated from the catalyst by simple operation, and highly expensive catalyst can be reused.

[Chem. 3]

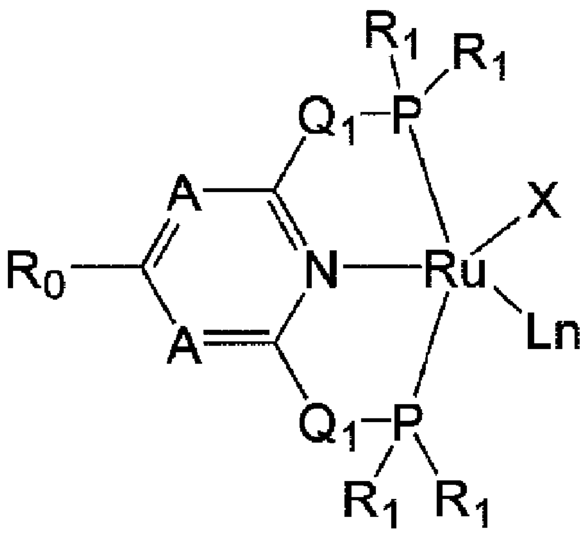

(1)

(In the formula (1), $R_0$ represents a hydrogen atom or an alkyl group,

$Q_1$ each independently represents $CH_2$, NH or O,
$R_1$ each independently represents an alkyl group or an aryl group (provided that when $Q_1$ represents NH or O, at least one of $R_1$ represents an aryl group),
A each independently represents CH, $CR_5$ or N, $R_5$ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.)

The $R_0$ in the formula (1) represents a hydrogen atom or an alkyl group. The alkyl group represented by $R_0$ includes a straight chain, branched or cyclic substituted or unsubstituted alkyl group.

The alkyl group represented by $R_0$ preferably includes an alkyl group having 1 to 30 carbon atoms, such as a methyl group, an ethyl group, an n-propyl group, an i-propyl group, a t-butyl group, an n-octyl group, an eicosyl group or a 2-ethylhexyl group. From the standpoint of easy procurement of raw materials, an alkyl group having 6 or fewer carbon atoms is preferred, and a methyl group is preferred.

The $R_0$ in the formula (1) is preferably a hydrogen atom or a methyl group.

$R_1$ in the formula (1) each independently represents an alkyl group or an aryl group, provided that when $Q_1$ represents NH or O, at least one of $R_1$ represents an aryl group.

The alkyl group represented by $R_1$ includes a straight chain, branched or cyclic substituted or unsubstituted alkyl group. The alkyl group represented by $R_1$ preferably includes an alkyl group having 1 to 30 carbon atoms, such as a methyl group, an ethyl group, an n-propyl group, an i-propyl group, a t-butyl group, an n-octyl group, an eicosyl group or a 2-ethylhexyl group. From the standpoint of catalyst activity, an alkyl group having 12 or fewer carbon atoms is preferred, and a t-butyl group is preferred.

The aryl group represented by $R_1$ includes a substituted or unsubstituted aryl group having 6 to 30 carbon atoms, such as a phenyl group, a p-tolyl group, a naphthyl group, an m-chlorophenyl group or an o-hexadecanoylaminophenyl group. An aryl group having 12 or fewer carbon atoms is preferred, and a phenyl group is more preferred.

A each independently represents CH, $CR_5$ or N, and $R_5$ represents an alkyl group, an aryl group, an aralkyl group, an amino group, a hydroxy group or an alkoxy group.

The alkyl group represented by $R_5$ includes a straight chain, branched or cyclic substituted or unsubstituted alkyl group. The alkyl group represented by $R_5$ preferably includes an alkyl group having 1 to 30 carbon atoms, such as a methyl group, an ethyl group, an n-propyl group, an i-propyl group, a t-butyl group, an n-octyl group, an eicosyl group or a 2-ethylhexyl group. From the standpoint of easy procurement of raw materials, an alkyl group having 12 or fewer carbon atoms is preferred, and a methyl group is preferred.

The aryl group represented by $R_5$ includes a substituted or unsubstituted aryl group having 6 to 30 carbon atoms, such as a phenyl group, a p-tolyl group, a naphthyl group, an m-chlorophenyl group or an o-hexadecanoylaminophenyl group. An aryl group having 12 or fewer carbon atoms is preferred, and a phenyl group is more preferred.

The aralkyl group represented by $R_5$ includes a substituted or unsubstituted aralkyl group having 30 or less carbon atoms, such as a trityl group, a benzyl group, a phenethyl group, a tritylmethyl group, a diphenylmethyl group or a naphthylmethyl group, and is preferably an aralkyl group having 12 or fewer carbon atoms.

The alkoxy group represented by $R_5$ preferably includes a substituted or unsubstituted alkoxy group having 1 to 30 carbon atoms, such as a methoxy group, an ethoxy group, an isopropoxy group, a t-butoxy group, an n-octyloxy group or a 2-methoxyethoxy group.

X represents a halogen atom and is preferably a chlorine atom.

n represents an integer of 0 to 3 and represents the number of ligands coordinating to ruthenium. From the standpoint of stability of the catalyst, n is preferably 2 or 3.

When more than one L are present, L each independently represents a neutral or anionic ligand.

The neutral ligand represented by L includes, for example, ammonia, carbon monoxide, phosphines (for example, triphenylphosphine or tris(4-methoxyphenyl)phosphine), phosphine oxides (for example, triphenyl phosphine oxide), sulfides (for example, dimethyl sulfide), sulfoxides (for example, dimethyl sulfoxide), ethers (for example, diethyl ether), nitriles (for example, p-methylbenzonitrile), and heterocyclic compounds (for example, pyridine, N,N-dimethyl-4-aminopyridine, tetrahydrothiophene or tetrahydrofuran), and is preferably triphenylphosphine.

The anionic ligand represented by L includes, for example, a hydride ion (hydrogen atom), a nitrate ion and a cyanide ion, and is preferably a hydride ion (hydrogen atom).

In the formula (1), A preferably represents CH, and $Q_1$ preferably represents NH.

Furthermore, n preferably represents 1 to 3, and L each independently preferably represents a hydrogen atom, carbon monoxide or triphenylphosphine.

The ruthenium complex represented by the formula (1) may be used singly alone and may be used as a mixture of two or more kinds.

The ruthenium complex represented by the formula (1) is preferably a ruthenium complex represented by the following formula (3).

[Chem. 4]

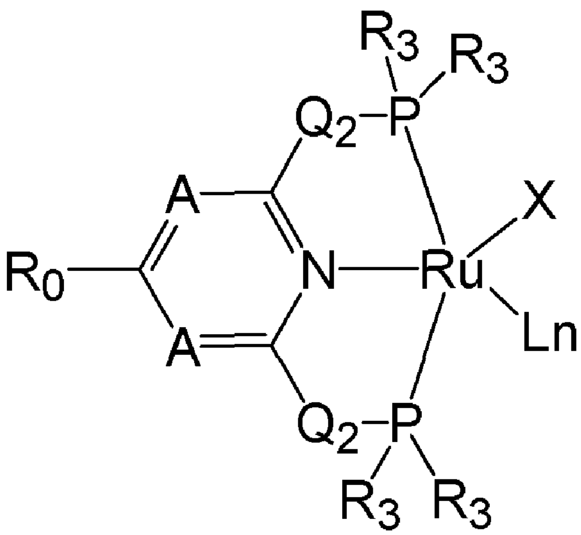

(3)

(In the formula (3), $R_0$ represents a hydrogen atom or an alkyl group,

$Q_2$ each independently represents NH or O,
$R_3$ each independently represents an aryl group,
A each independently represents CH, $CR_5$ or N, $R_5$ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.)

$R_0$, A, $R_5$, X, n and L in the formula (3) are synonymous with $R_0$, A, $R_5$, X, n and L in the formula (1), respectively, and preferred ranges thereof are also the same.

The aryl groups represented by $R_3$ in the formula (3) are each synonymous with the aryl group represented by $R_1$ in the formula (1), and the preferred ranges thereof are also the same.

Regarding the ruthenium complexes represented by the formula (1) and formula (3), ruthenium complexes produced by known methods can be used. As the known methods, for example, the methods described in E. Pidko et al., ChemCatChem 2014, 6, 1526-1530, and the like can be used.

The ruthenium complexes represented by the formula (1) and formula (3) may form stereoisomers due to coordination of a ligand or conformation, but may be a mixture of these stereoisomers or may be a single pure isomer.

Specific examples of the ruthenium complex, the ruthenium complexes represented by the formula (1) and formula (3), and the ligand include compounds listed below.

In the compounds listed below, tBu represents a tertiary butyl group, and Ph represents a phenyl group.

[Chem. 5]

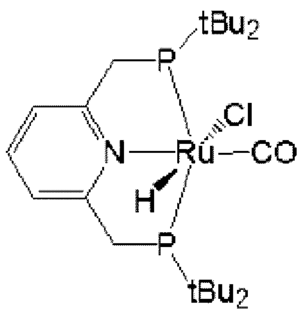

1

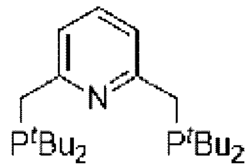

A

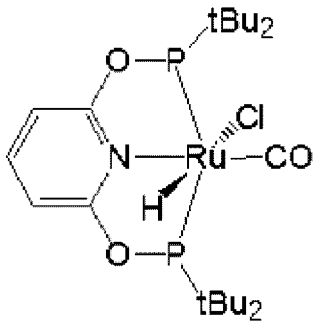

2

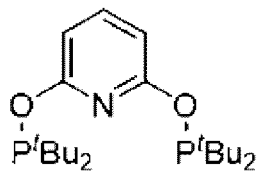

B

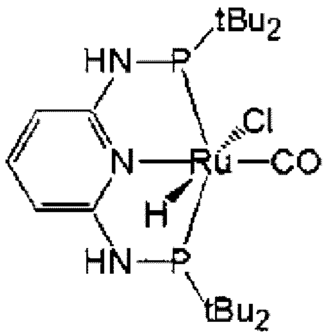

3

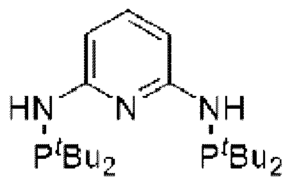

C

[Chem. 6]

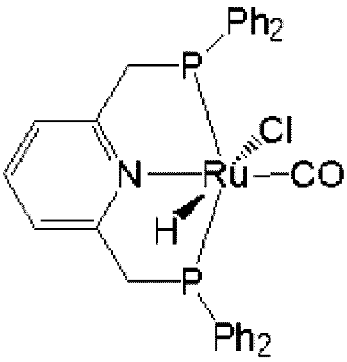

5

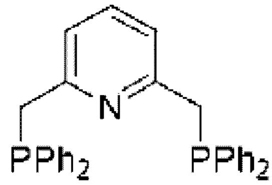

E

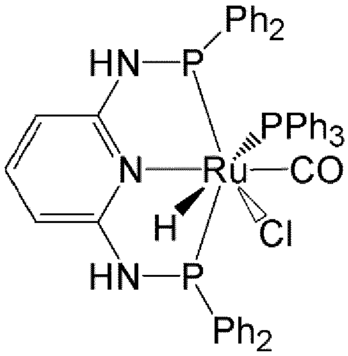

7

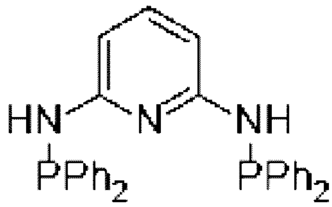

G

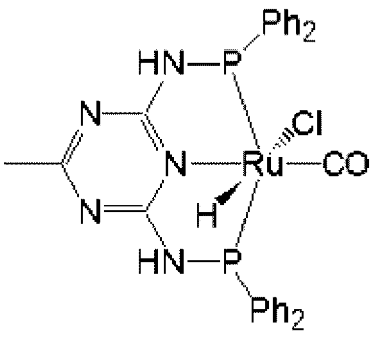

8

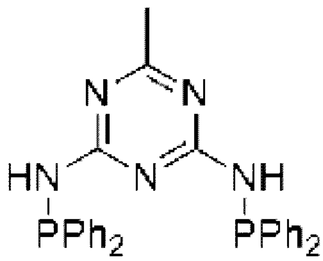

H

The amount of the ruthenium complex used as the catalyst is not particularly limited so long as an alkaline earth metal formate can be produced. The amount of the ruthenium complex used as the catalyst is preferably 0.1 μmol or more, more preferably 0.5 μmol or more, and still more preferably 1 μmol or more, per 1 L of the solvent, in order to sufficiently express the catalyst function. Furthermore, the amount of the ruthenium complex is preferably 1 mol or less, more preferably 10 mmol or less, and still more preferably 1 mmol or less, from the standpoint of cost. When two or more kinds of ruthenium complexes are used, the total amount of those used needs to be in the above range.

When the homogeneous catalyst used in the method for producing an alkaline earth metal formate according to an embodiment of the invention is a metal complex catalyst, the ligand for forming the complex is preferably present in excess in the reaction system. Therefore, the ligand of the metal complex catalyst used is preferably further added to the reaction system.

For example, when the above ruthenium complex is used as the homogeneous catalyst, the ligand for forming the ruthenium complex is preferably further added into the reaction mixture together with the above ruthenium complex. For example, when the homogeneous catalyst is a ruthenium complex represented by the formula (1), a ligand represented by the following formula (4) is preferably further added.

[Chem. 7]

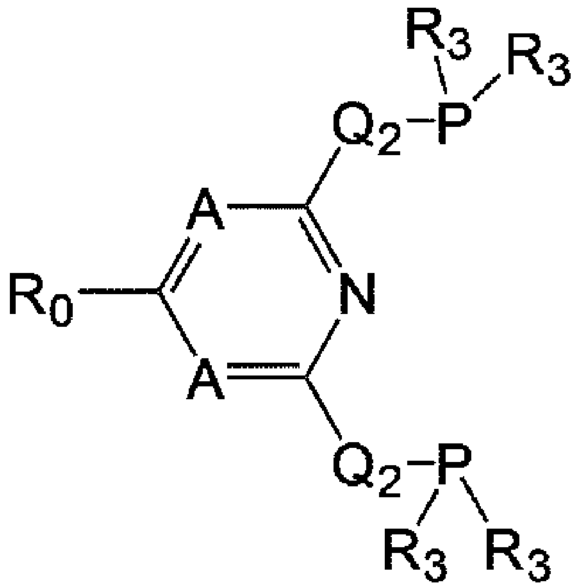

(4)

(In the formula (4), $R_0$ represents a hydrogen atom or an alkyl group,

$Q_2$ each independently represents NH or O,
$R_3$ each independently represents an aryl group,
A each independently represents CH, $CR_5$ or N, and $R_5$ represents an alkyl group, an aryl group, an aralkyl group, an amino group, a hydroxy group or an alkoxy group.)

$R_0$, $Q_2$, $R_3$, A and $R_5$ in the formula (4) are synonymous with $R_0$, $Q_2$, $R_3$, A, and $R_5$ in the formula (3), respectively, and preferred ranges thereof are also the same.

By adding the ligand for forming a complex to the reaction system in excess, even when the ligand is oxidized and deteriorated by oxygen and impurities included in the system, the deteriorated ligand and the added ligand are exchanged to restore the catalyst function, and therefore, stability of the catalyst can be improved.

Addition of the ligand represented by the above formula (4) into the reaction mixture may be carried out when the reaction mixture is prepared or may be carried out in the middle of the reaction. However, from the standpoint of process management, the addition is preferably carried out when the reaction mixture is prepared.

(Phase transfer catalyst)

The method for producing an alkaline earth metal formate according to an embodiment of the invention requires to conduct the reaction in a two-phase system. Therefore, a phase transfer catalyst may be used in order to smoothly perform the transfer of a substance between two phases. The phase transfer catalyst includes, for example, a quaternary ammonium salt, a quaternary phosphate, a macrocyclic polyether such as a crown ether, a nitrogen-containing macrocyclic polyether such as a cryptand, a nitrogen-containing chain polyether, polyethylene glycol and an alkyl ether thereof. Above all, a quaternary ammonium salt is preferred from the standpoint that mass transfer between an aqueous solvent and an organic solvent is easy even under mild reaction conditions.

The quaternary ammonium salt includes, for example, methyltrioctylammonium chloride, benzyltrimethylammonium chloride, trimethylphenylammonium bromide, tributylammonium tribromide, tetrahexylammonium hydrogen sulfate, decyltrimethylammonium bromide, diallyldimethylammonium chloride, dodecyltrimethylammonium bromide, dimethyldioctadecylammonium bromide, tetraethylammonium tetrafluoroborate, ethyltrimethylammonium iodide tris(2-hydroxyethyl)methylammonium hydroxide, tetramethylammonium acetate, tetramethylammonium bromide, and tetraethylammonium iodide. methyltrioctylammonium chloride is preferred.

The amount of the phase transfer catalyst used is not particularly limited so long as an alkaline earth metal formate can be produced. The amount of the phase transfer catalyst used is preferably 0.1 mmol or more, more preferably 0.5 mmol or more and still more preferably 1 mmol or more, per 1 liter of the solvent for the purpose of efficiently supporting the transfer of a carbonate or a hydrogen carbonate. Furthermore, from the standpoint of cost, the amount is preferably 1 mol or less, more preferably 500 mmol or less and still more preferably 100 mmol or less. When two or more kinds of the phase transfer catalysts are used, the total amount of those needs to be in the above range.

(Carbon dioxide and hydrogen)

As hydrogen used in an embodiment of the invention, either a hydrogen gas cylinder or liquid hydrogen can be used. As a hydrogen supply source, for example, hydrogen generated during a smelting process of iron manufacture, hydrogen generated during a soda manufacturing process, and the like can be used. Furthermore, hydrogen generated from electrolysis of water can be used.

Carbon dioxide used in an embodiment of the invention may be pure carbon dioxide gas or may be a mixed gas containing a component other than carbon dioxide. Carbon dioxide gas and other gas may be separately introduced, and a mixed gas may be formed beforehand and introduced.

The component other than carbon dioxide includes an inert gas such as nitrogen or argon, water vapor, and any optional component contained in an exhaust gas or the like.

As the carbon dioxide, a carbon dioxide gas cylinder, liquid carbon dioxide, supercritical carbon dioxide, dry ice, and the like can be used.

Hydrogen gas and carbon dioxide gas may be introduced into the reaction system each alone or may be introduced as a mixed gas.

The proportions of hydrogen and carbon dioxide used are preferably such that the proportions are equal amounts on a molar basis or hydrogen is in excess.

When a hydrogen cylinder is used as the hydrogen used in the method for producing an alkaline earth metal formate according to an embodiment of the invention, the pressure is preferably 0.1 MPa or more, more preferably 0.2 MPa or more and still more preferably 0.5 MPa or more, from the standpoint of sufficiently securing reactivity. Furthermore, the pressure is preferably 50 MPa or less, more preferably 20 MPa or less and still more preferably 10 MPa or less, from the standpoint that facilities are liable to become large.

The pressure of carbon dioxide used in the method for producing an alkaline earth metal formate according to an embodiment of the invention is preferably 0.1 MPa or more, more preferably 0.2 MPa or more and still more preferably 0.5 MPa or more, from the standpoint of sufficiently securing reactivity. Furthermore, the pressure is preferably 50 MPa or less, more preferably 20 MPa or less and still more preferably 10 MPa or less, from the standpoint that facilities are liable to become large.

Hydrogen gas and carbon dioxide gas may be introduced into a catalyst solution by bubbling (blowing). Furthermore, after introducing a gas including hydrogen gas and carbon dioxide gas, the catalyst solution, hydrogen gas and carbon dioxide gas may be stirred by stirring with a stirring device or by rotating the reaction vessel.

A method for introducing carbon dioxide, hydrogen, a catalyst, a solvent and the like that are used for the reaction into the reaction vessel is not particularly limited. All of the raw materials may be introduced at once, a part or all of the raw materials may be introduced stepwise, or a part or all of the raw materials may be introduced continuously. Furthermore, the method may be an introduction method combining those methods.

(Hydrogen carbonate and carbonate)

In the method for producing an alkaline earth metal formate according to an embodiment of the invention, a hydrogen carbonate or carbonate of an alkaline earth metal is used.

The hydrogen carbonate of an alkaline earth metal includes, for example, calcium hydrogen carbonate, strontium hydrogen carbonate, barium hydrogen carbonate, and radium hydrogen carbonate, and calcium hydrogen carbonate is preferred from the standpoint of high solubility in water.

The alkaline earth metal carbonate includes, for example, calcium carbonate, strontium carbonate, barium carbonate, and radium carbonate.

The alkaline earth metal in the carbonate or hydrogen carbonate of the alkaline earth metal is preferably calcium.

The base concentration in the reaction liquid is preferably 0.1 mol/L or more.

The base concentration in the reaction liquid is preferably 0.1 mol/L or more, more preferably 0.5 mol/L or more, and still more preferably 1 mol/L or more, from the standpoint of increasing the maximum amount of production of the formate. Furthermore, in order to prevent that the amount of the precipitated salt derived from the base is excessively increased to make stirring of the reaction unfavorable, the base concentration is preferably 30 mol/L or less, more

preferably 25 mol/L or less, and still more preferably 20 mol/L or less.

A hydrogen carbonate and a carbonate of an alkaline earth metal are preferably used so that the base concentration in the reaction liquid is in the above range.

(Reaction conditions)

The reaction conditions in the method for producing an alkaline earth metal formate according to an embodiment of the invention are not particularly limited, and the reaction conditions can be appropriately changed during the reaction process. The form of the reaction vessel used for the reaction is not particularly limited.

The reaction temperature is not particularly limited. However, to efficiently proceed the reaction, the temperature is preferably 30°C or higher, more preferably 40°C or higher and still more preferably 50°C or higher. Furthermore, from the standpoint of energy efficiency, the reaction temperature is preferably 200°C or lower, more preferably 150°C or lower and still more preferably 100°C or lower.

The reaction time is not particularly limited. However, for example, the reaction time is preferably 0.5 hours or more, more preferably 1 hour or more, and still more preferably 2 hours or more, from the standpoint of sufficiently securing the amount of the formate formed. Furthermore, the reaction time is preferably 24 hours or less, more preferably 20 hours or less, and still more preferably 18 hours or less, from the standpoint of cost.

The concentration of the formate (concentration of the formate in the aqueous phase) formed by the first step is preferably 0.1 mol/L or more, more preferably 0.5 mol/L or more, and still more preferably 1 mol/L or more, in order to increase the TON and produce the formate in high yield and with excellent productivity. Furthermore, in order to simplify the production process by producing the formate in a dissolved state, the concentration of the formate is preferably 30 mol/L or less, more preferably 25 mol/L or less, and still more preferably 20 mol/L or less.

After completion of the reaction in the first step, the homogeneous catalyst is separated and recovered from the reaction solution by conducting ordinary separation, posttreatment, refinement operation and the like, and the alkaline earth metal salt can be isolated.

(Second step)

The second step is a step of separating a solution containing the homogeneous catalyst from the reaction solution obtained by the first step, by partitioning.

The method for producing an alkaline earth metal formate according to an embodiment of the invention preferably further includes the second step.

In an embodiment of the invention, since the reaction proceeds in a two-phase system, the alkaline earth metal formate formed by the reaction is eluted into the aqueous phase. Therefore, when a homogeneous catalyst that is easily dissolved in an organic solvent is used, the organic phase and the aqueous phase are partitioned to separate the organic phase as a solution containing the homogeneous catalyst, and the homogeneous catalyst can be recovered.

The method for partitioning is not particularly limited, and ordinary methods are used. For example, partitioning can be conducted by extracting either the organic phase or the aqueous phase under an inert gas using the drain port of the reaction vessel, an installed pump or the like.

The homogeneous catalyst separated and recovered by the second step can be reused in the first step. For example, the solution containing the homogeneous catalyst separated may be reused in the first step as it is, and the concentration of the homogeneous catalyst may be adjusted or the homogeneous catalyst may be isolated and recovered, by operations such as concentration and refinement.

## EXAMPLES

The invention is described in detail below by reference to Examples and Comparative Examples. However, it should be understood that the invention is not limited to those Examples.

[Synthesis of catalyst]

(Synthesis Example 1) Synthesis of Ru catalyst 1

Ru catalyst 1 was synthesized by the following operation.

40 mg (0.1 mmol) of a ligand A shown below was added to a THF (tetrahydrofuran) (5 ml) suspension of 95.3 mg (0.1 mmol) of $[RuHCl(PPh_3)_3(CO)]$ in an inert atmosphere, the resulting mixture was stirred and heated at 65°C for 3 hours to conduct a reaction. Thereafter, the resulting reaction mixture was cooled to room temperature (25°C).

A yellow solution obtained was filtered, and the filtrate was evaporated to dryness under a vacuum. Yellow

residual oil obtained was dissolved in THF (1 mL), hexane (10 mL) was slowly added to the resulting solution to precipitate a yellow solid, and the solid was filtered and dried under vacuum. Thus, Ru catalyst 1 (55 mg, 97%) as yellow crystals was obtained. In the Ru ruthenium catalyst 1 and ligand A shown below, tBu indicates a tertiary butyl group.

[Chem. 8]

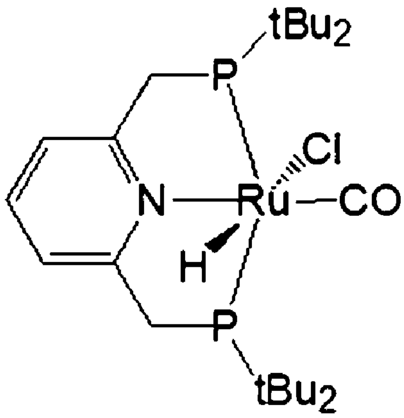

1

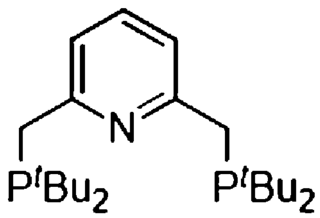

A

$^{31}P\{^{1}H\}(C_6D_6)$:90.8 (s), $^{1}H$ $(C_6D_6)$:-14.54 (t, 1H, $J$=20.0Hz), 1.11 (t, 18H, $J$=8.0Hz), 1.51 (t, 18H, $J$=8.0Hz) ,2.88 (dt, 2H, J=16.0Hz, J=4.0Hz), 3.76 (dt, 2H, J=16.0Hz, J=4.0Hz), 6.45 (d, 2H, $J$=8.0Hz), 6.79 (t, 1H, J=8.0Hz).
$^{13}C\{^{1}H\}$ NMR $(C_6D_6)$:29.8 (s), 30.7 (s), 35.2 (t, J=9.5Hz), 37.7 (t, J=6.0Hz), 37.9 (t, J=6.5Hz), 119.5 (t, J=4.5Hz), 136.4 (s), 163.4 (t, J=5.0Hz), 209.8 (s).

(Synthesis Example 2) Synthesis of Ru catalyst 7

Ru catalyst 7 was synthesized by the following operation.

142.6 mg of a ligand G and 284.6 mg of $[RuHCl(PPh_3)_3(CO)]$ were mixed with 5 mL of benzene in an inert atmosphere, and the resulting suspension was refluxed one night. A yellow precipitate formed was collected on a filter and cleaned with 5 mL of ether 4 times.

The precipitate was dried in a vacuum, and 154.0 mg of Ru catalyst 7 was obtained.

In the Ru catalyst 7 and ligand G shown below, Ph indicates a phenyl group.

[Chem. 9]

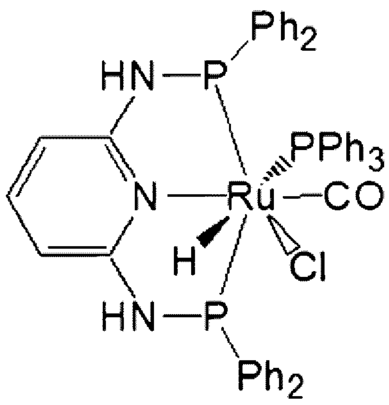

7

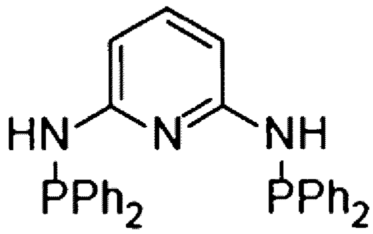

G

$^{31}P\{^{1}H\}$NMR($CDC_3$): 95.58 (br, s), 29.71 (s).$^{1}H$ NMR (400MHz, $CD_2Cl_2$) δ9.92 (s, 2H), 8.11 (q, J=6.6Hz, 4H), 7.38-7.24 (m, 4H), 7.20 (t, J=7.5Hz, 3H), 7.16-7.04 (m, 4H), 7.04-6.92 (m, 14H), 6.87 (td, J=7.6, 2.1Hz, 6H), 6.51 (d, J=8.0Hz, 1H), 6.61 (d, J=8.0Hz, 2H),-7.22 (dt, J=89.2, 23.1Hz, 1H).

<Example 1>

(Calcium formate forming reaction)

5 mmol of calcium carbonate was added in 5 mL of water in a glass vial equipped with a stirring rod in a glovebox under an inert gas, and thereafter 0.6 μmol of the Ru catalyst 7 and 270 μmol of methyltrioctylammonium chloride were added to 5 mL of toluene. Thereafter, the vial was placed in an autoclave, and the autoclave was sealed and taken out of the glovebox.

The autoclave was heated to 90°C while being stirred. When the temperature reached the target temperature, the autoclave was pressurized to 4 MPa by charging a gas containing 50 vol% of hydrogen and 50 vol% of carbon dioxide into the autoclave. The reaction mixture was stirred for 4.5 hours, thereafter the reaction mixture was cooled with an ice bath, and pressure was carefully released. An organic phase (solution containing the homogeneous catalyst) of the solution after the reaction was separated, and unreacted calcium carbonate that was precipitated in the aqueous phase was removed to obtain an aqueous solution containing calcium formate.

The homogeneous catalyst and calcium carbonate could be separated by the above operation.

(Method for quantifying calcium formate in aqueous solution)

100 μL of the aqueous solution containing calcium formate was collected and dissolved in 500 μL of deuterated water, 300 μL of dimethyl sulfoxide was added as an internal standard, and then $^{1}H$ NMR was measured. The molar amount (mol) X of calcium formate (molar amount (mol) of calcium formate formed by the reaction) included in the solution was calculated by the following formula. $X = (W/M) \times (Ia \times Ib/R) \times (A/B)$

W: Amount (g) of dimethyl sulfoxide used for quantification of calcium formate,
M: Molecular weight of dimethyl sulfoxide,
R: Ratio of the number of protons of dimethyl sulfoxide to the number of protons of calcium formate,
Ia: Proton NMR integration value of formate ions,
Ib: NMR Integration value of dimethyl sulfoxide,
A: Mass (g) of sample solution, and
B: Mass (g) of solution used for quantification.

Here, since W is 0.33, M is 78.13, and R is 6, the following is obtained.

$$X = 0.0007 \times Ia \times Ib \times (A/B)$$

(Calculation of turnover frequency (TOF) of catalyst)

Calculation of the "TOF of catalyst" described in Table 1 was determined by dividing the molar amount (mol) of calcium formate formed in the reaction by 0.0006 (mol), which was the molar amount of the catalyst used for the reaction, to calculate the TON of the catalyst and dividing the calculated TON by 4.5, which was the reaction time (hr).

<Comparative Example 1>

The description of Table 2. Entry 6 (reaction temperature 60°C, reaction pressure 4 MPa (hydrogen:carbon dioxide = 1:1), reaction time 20 hours) described in Non-Patent Literature Green Chem., 2020, 22, 4995-5001 was adopted as Comparative Example 1. The TON of the catalyst under the conditions was 1750, and the TOF of the catalyst was calculated by dividing this TON by 20 as the reaction time (hr).

<Comparative Example 2>

The description of Fig. 6 (reaction temperature 50°C, reaction pressure 5 MPa (hydrogen:carbon dioxide = 4:1), reaction time 24 hours) described in Non-Patent Literature Journal of Molecular Catalysis A: Chemical 224 (2004), 87-91, was adopted as Comparative Example 2. Since the concentration of calcium formate was formed up to about 220 mmol/L with respect to 1 mmol/L of the concentration of the Rh catalyst under the conditions, the TON of the catalyst was calculated by dividing the concentration of calcium formate formed by the concentration of the Rh catalyst, and the TON was divided by 24 as the reaction time (hr) to calculate the TOF.

The above Examples and Comparative Examples are described in Table 1.

[Table 1]

Table 1

| | Example 1 | Comparative Example 1 | Comparative Example 2 |
|---|---|---|---|
| Catalyst system/reaction system | Homogeneous Ru catalyst/two-phase system | Heterogeneous Ru catalyst/one-phase system | Homogeneous Rh catalyst/one-phase system |
| Solvent | Water = 5 mL<br>Toluene = 5 mL | Water = 20 mL | Water = 10 mL |
| Kind of base | CaCO3 | CaCO3 | CaCO3 |
| Reaction temperature (°C) | 90 | 60 | 50 |
| Reaction pressure (MPa) | 4 (Hydrogen:carbon dioxide = 1: 1) | 4 (Hydrogen:carbon dioxide = 1:1) | 5 (Hydrogen:carbon dioxide = 4:1) |
| Reaction time (hr) | 4.5 | 20 | 24 |
| TOF of catalyst | 130 | 88 | 9.2 |
| Separation of calcium formate and homogeneous catalyst | Possible | Possible | Impossible |

It was found that Example 1 in which the alkaline earth metal formate was produced using the production method according to an embodiment of the invention showed high TOF and had excellent production efficiency for the alkaline earth metal formate as compared with Comparative Examples 1 and 2.

INDUSTRIAL APPLICABILITY

According to the invention, a method for producing an alkaline earth metal formate, in which an alkaline earth metal formate can be produced in high yield and with excellent productivity, can be provided.

The invention was described in detail and with reference to specific embodiments. However, it is obvious to

those ordinarily skilled in the art that various modifications and alterations can be made without departing from the spirit and scope of the invention.

The invention is based on Japanese Patent Application (Japanese Patent Application No. 2020-148562) filed on September 3, 2020, Japanese Patent Application (Japanese Patent Application No. 2021-021223) filed on February 12, 2021, Japanese Patent Application (Japanese Patent Application No. 2021-021224) filed on February 12, 2021, Japanese Patent Application (Japanese Patent Application No. 2021-021225) filed on February 12, 2021, Japanese Patent Application (Japanese Patent Application No. 2021-079887) filed on May 10, 2021, and Japanese Patent Application (Japanese Patent Application No. 2021-083416) filed on May 17, 2021, the disclosures of which are incorporated herein by reference.

## Claims

**1.** A method for producing an alkaline earth metal formate, the method comprising:

a first step of reacting hydrogen and carbon dioxide with a carbonate or hydrogen carbonate of an alkaline earth metal
using a homogeneous catalyst
in the presence of a solvent
in a two-phase system in which an organic phase and an aqueous phase are present in a separated state in the solvent
to produce a formate of an alkaline earth metal.

**2.** The method for producing an alkaline earth metal formate according to claim 1, wherein the method further includes a second step of separating a solution including the homogeneous catalyst from the reaction solution obtained by the first step, by partitioning.

**3.** The method for producing an alkaline earth metal formate according to claim 1 or 2, wherein the alkaline earth metal is calcium.

**4.** The method for producing an alkaline earth metal formate according to any one of claims 1 to 3, wherein the homogeneous catalyst contains at least one metal selected from ruthenium, iridium, iron, nickel, and cobalt.

**5.** The method for producing an alkaline earth metal formate according to any one of claims 1 to 4, wherein the organic phase contains at least one selected from toluene, dioxane, tetrahydrofuran, ethyl acetate, methylcyclohexane, and cyclopentyl methyl ether.

**6.** The method for producing an alkaline earth metal formate according to any one of claims 1 to 5, wherein the homogeneous catalyst is a metal complex catalyst, and a ligand of the metal complex catalyst is further added.

**7.** The method for producing an alkaline earth metal formate according to any one of claims 1 to 5, wherein the homogeneous catalyst is at least one selected from a ruthenium complex represented by the following formula (1), a tautomer or stereoisomer thereof, and a salt compound of the ruthenium complex, tautomer or stereoisomer:

[Chem. 1]

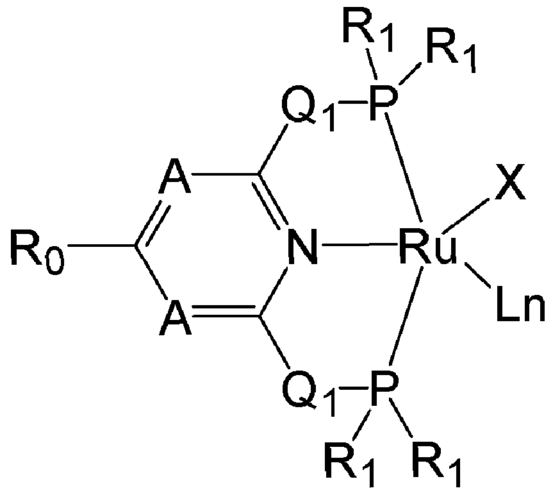

(1)

wherein $R_0$ represents a hydrogen atom or an alkyl group,

$Q_1$ each independently represents $CH_2$, NH or O,
$R_1$ each independently represents an alkyl group or an aryl group (provided that when $Q_1$ represents NH or O, at least one of $R_1$ represents an aryl group),
A each independently represents CH, $CR_5$ or N, $R_5$ represents an alkyl group, an aryl group, an aralkyl group, an amino group, a hydroxy group or an alkoxy group,
X represents a halogen atom,
n represents 0 to 3, and
when more than one L are present, L each independently represents a neutral or anionic ligand.

**8.** The method for producing an alkaline earth metal formate according to claim 7, wherein a ligand represented by the following formula (4) is further added:

[Chem. 2]

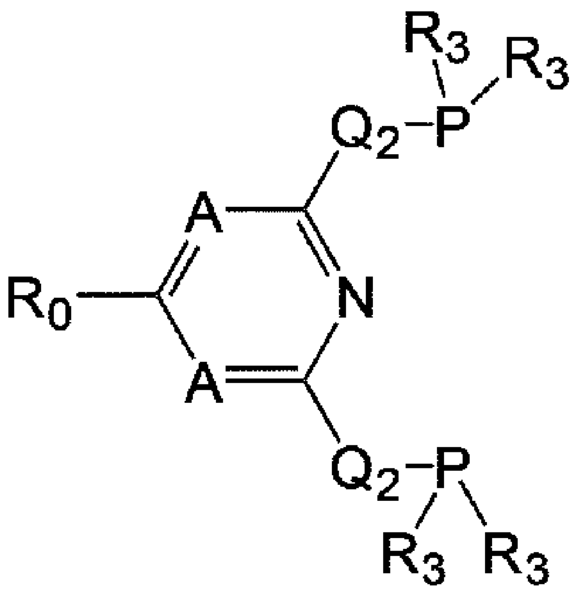

(4)

wherein $R_0$ represents a hydrogen atom or an alkyl group,

$Q_2$ each independently represents NH or O,
$R_3$ each independently represents an aryl group,
A each independently represents CH, $CR_5$ or N, and $R_5$ represents an alkyl group, an aryl group, an aralkyl group, an amino group, a hydroxy group or an alkoxy group.

**9.** The method for producing an alkaline earth metal formate according to any one of claims 1 to 8, wherein a quaternary ammonium salt is further used as a phase transfer catalyst in the first step.

## INTERNATIONAL SEARCH REPORT

International application No.
**PCT/JP2021/031793**

**A. CLASSIFICATION OF SUBJECT MATTER**

***C07C 51/00***(2006.01)i; ***C07C 51/02***(2006.01)i; ***C07C 53/02***(2006.01)i; ***C07C 53/06***(2006.01)i; ***C07F 15/00***(2006.01)i; ***C07F 19/00***(2006.01)i; ***C09K 3/00***(2006.01)i; ***C07B 61/00***(2006.01)i; ***C07F 9/50***(2006.01)i; ***B01J 31/02***(2006.01)i; ***B01J 31/22***(2006.01)i; ***B01J 31/24***(2006.01)i
FI: C07C51/00; C07C53/02; B01J31/24 M; C07F15/00 A; C07C53/06; B01J31/24 Z; B01J31/02 102Z; C07C51/02; C07B61/00 300; C09K3/00 102; C07F19/00; C07F9/50; B01J31/22 M; B01J31/02 102M

According to International Patent Classification (IPC) or to both national classification and IPC

**B. FIELDS SEARCHED**

Minimum documentation searched (classification system followed by classification symbols)

C07C51/00; C07C51/02; C07C53/02; C07C53/06; C07F15/00; C07F19/00; C09K3/00; C07B61/00; C07F9/50; B01J31/02; B01J31/22; B01J31/24

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | GUNASEKAR, Gunniya Hariyanandam et al. Eco-friendly upconversion of limestone into value added calcium formate. Green Chemistry. 29 June 2020, vol. 22, pages 4995-5001 fig. 1, table 1 | 1-6, 9 |
| A | | 7, 8 |
| Y | JOSZAI, Istvan et al. Hydrogenation of aqueous mixtures of calcium carbonate and carbon dioxide using a water-soluble rhodium (I)-tertiary phosphine complex CATalyst. Journal of Molecular Catalysis A: Chemical 224 (2004). 2004, vol. 224, pages 87-91 entire text, 3. Results and discussion etc. | 1-6, 9 |
| A | | 7, 8 |
| Y | JP 2015-505857 A (BASF SE) 26 February 2015 (2015-02-26) claim 1, paragraphs [0008], [0013] | 1-6, 9 |
| A | | 7, 8 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 October 2021** | **12 October 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
PCT/JP2021/031793

C. DOCUMENTS CONSIDERED TO BE RELEVANT

| Category* | Citation of document, with indication, where appropriate, of the relevant passages | Relevant to claim No. |
|---|---|---|
| A | TANAKA, Ryo et al. Catalytic Hydrogenation of Carbon Dioxide Using Ir (III)-Pincer Complexes. Journal of the American Chemical Society. 23 September 2009, vol. 131 (40), pages 14168-14169<br>scheme 1 | 1-9 |
| A | BERTINI, Federica et al. Efficient and Mild Carbon Dioxide Hydrogenation to Formate Catalyzed by Fe (II) Hydrido Carbonyl Complexes Bearing 2, 6- (Diaminopyridyl) diphosphine Pincer Ligands. ACS Catalysis. 30 March 2016, vol. 6 (5), pages 2889-2893<br>scheme 1, scheme 2 | 1-9 |

Form PCT/ISA/210 (second sheet) (January 2015)

**INTERNATIONAL SEARCH REPORT**
**Information on patent family members**

International application No.
**PCT/JP2021/031793**

| Patent document cited in search report | Publication date (day/month/year) | Patent family member(s) | Publication date (day/month/year) |
|---|---|---|---|
| JP 2015-505857 A | 26 February 2015 | WO 2013/092157 A1<br>claim 1, pages 3-4<br>EP 2794540 A1<br>CN 103998409 A<br>KR 10-2014-0105577 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H11286466 A
- JP 5896539 B
- JP 2020148562 A
- JP 2021021223 A
- JP 2021021224 A
- JP 2021021225 A
- JP 2021079887 A
- JP 2021083416 A


### Non-patent literature cited in the description


- *Chem. Rev.,* 2017, vol. 117, 9804-9838
- *Chem. Rev.,* 2018, vol. 118, 372-433
- **E. PIDKO et al.** *ChemCatChem,* 2014, vol. 6, 1526-1530
- *Green Chem.,* 2020, vol. 22, 4995-5001
- Journal of Molecular Catalysis A: Chemical. 2004, vol. 224, 87-91